# FASCICULE DE BREVET EUROPEEN

(11) **EP 1 163 008 B1**
(45) Date de publication et mention de la délivrance du brevet: **13.08.2003**
(21) Numéro de dépôt: 00912734.1
(22) Date de dépôt: 23.03.2000
(51) Int. Cl.: A61K 47/26, A61K 39/02, A61K 39/09, A61K 39/095, A61K 39/102, A61K 39/385, A61P 31/04

(54) **UTILISATION DE TREHALOSE POUR STABILISER UN VACCIN LIQUIDE**
VERWENDUNG VON THREALOSE ZUR STABILISIERUNG VON EINEM FLÜSSIGEN IMPFSTOFF
USE OF TREHALOSE FOR STABILISING A LIQUID VACCINE

(30) Priorité: 23.03.1999 FR 9903765
(43) Date de publication de la demande: 19.12.2001
(73) Titulaire: Aventis Pasteur, 69007 Lyon (FR)
(72) Inventeur: LENTSCH GRAF, Sandrine, F-69004 Lyon (FR); CARTIER, Jean-René, F-69005 Lyon (FR)
(74) Mandataire: Kerneis, Danièle
(86) Numéro de dépôt international: FR0000730
(87) Numéro de publication internationale: WO00056365

(56) Documents cités:
- WO-A-86/06635
- WO-A-95/33488
- WO-A-99/12568
- US-A- 4 206 200
- PAIVA C & PANEK A: "Biotechnological applications of the disaccharide trehalose" BIOTECHNOLOGY ANNUAL REVIEW, vol. 2, 1996, pages 293-314, XP000870408

## Description

L'invention est relative au domaine des vaccins. Plus particulièrement, l'invention concerne un procédé de stabilisation de compositions vaccinales liquides comprenant, parmi leurs antigènes, au moins un polysaccharide lié à une protéine porteuse.

On connaît, dans l'art antérieur, de telles compositions vaccinales, dont certains antigènes, pour être immunogènes, doivent être liés à des protéines porteuses. C'est notamment le cas des compositions destinées à la vaccination contre les infections provoquées par la bactérie Haemophilus influenzae de type b, qui comprennent, à titre d'antigène vaccinal, le polysaccharide capsulaire de la bactérie ou Polyribosylribitol Phosphate (PRP) couplé à l'anatoxine tétanique T. De telles compositions vaccinales ont tendance à perdre de leur immunogénicité, et donc de leur efficacité au cours du temps. Pour pallier cet inconvénient, la solution généralement proposée dans l'art antérieur est la lyophilisation. Cette solution, satisfaisante d'un point de vue du résultat obtenu en matière de conservation de l'immunogénicité, présente cependant l'inconvénient d'alourdir le procédé de fabrication, et donc d'en accroître le coût. En outre, au moment de l'administration du vaccin, il est nécessaire d'effectuer une opération supplémentaire de reprise du lyophilisat par un liquide stérile, ce qui d'une part représente une contrainte supplémentaire pour le praticien et d'autre part, comporte, comme toute manipulation, le risque d'être mal effectuée. Il est donc souhaitable de trouver une autre solution au problème de la perte d'immunogénicité au cours du temps des antigènes polysaccharidiques liés à une protéine porteuse lorsqu'ils sont présents dans une composition vaccinale liquide.

A cette fin, l'invention propose un procédé de stabilisation d'une composition vaccinale liquide comprenant au moins un antigène constitué par un polysaccharide lié à une protéine porteuse, caractérisé en ce qu'il consiste à ajouter à la composition vaccinale du tréhalose.

Ainsi, on obtient une composition vaccinale qui, bien que liquide, conserve son caractère immunogène au cours du temps, même lorsqu'elle est conservée à température ambiante.

Le procédé selon l'invention présente l'avantage d'être simple et rapide, ce qui en fait un procédé de choix pour un industriel.

De nombreux autres avantages de la présente invention apparaîtront à la lecture de la description détaillée qui suit.

Le procédé de stabilisation d'une composition vaccinale liquide est charactérisé en ce que ladite composition vaccinale peut être une composition monovalente, c'est-à-dire qu'elle est destinée à la protection contre une seule maladie, ou plurivalente, c'est-à-dire qu'elle est destinée à protéger l'individu à qui elle a été administrée, contre plusieurs maladies. Dans tous les cas, au moins une des valences vaccinales est représentée par un antigène polysaccharidique lié à une protéine porteuse. Parmi les antigènes polysaccharidiques susceptibles d'entrer dans la composition d'un vaccin et d'être stabilisés selon l'invention, on peut citer les polysaccharides présents dans les capsules de bactéries, les polysaccharides présents dans les parois de bactéries Gram négatif ou encore les polysaccharides présents dans les parois de champignons. Ainsi, il est possible d'utiliser les polysaccharides rencontrés dans les microorganismes suivants : Pseudomonas (par exemple P. aeruginosa), Staphylococcus, Streptococcus (par exemple S. pneumoniae), Klebsiella (par exemple K. pneumonia), Salmonella (par exemple S. typhi et S. paratyphi), Escherichia coli, Neisseria (par exemple N. meningitidis), Shigella (par exemple S. dysenteria, somnei ou flexneri), Haemophilus (par exemple H. influenzae de type b), Moraxella, Vibrio cholerae, Mycobacterium tuberculosis, Candida, Cryptococcus neoformans ainsi que Hansenula.

La présente invention a montré tout son intérêt pour un procédé de stabilisation des compositions vaccinales comprenant le polysaccharide capsulaire de Haemophilus influenzae type b ou Polyribosylribitol Phosphate.

Les polysaccharides utilisés généralement en tant qu'antigènes vaccinaux présentent généralement la caractéristique d'être T-indépendants, c'est-à-dire notamment que la mémoire du système immunitaire vis-à-vis de tels antigènes est faible et que ces polysacharides ne sont généralement pas immunogènes chez les jeunes enfants. Afin de les rendre T-dépendants, il est usuel de les associer à des protéines porteuses (par protéine, au sens de la présente invention, on inclut également les peptides ou polypeptides) afin d'obtenir un conjugué polysaccharide-protéine porteuse. Ces protéines sont notamment celles utilisées habituellement dans le domaine des vaccins : anatoxine diphtérique, anatoxine tétanique, forme mutante non-toxique CRM₁₉₇ de l'anatoxine diphtérique, protéine de membrane externe de type 1 (OMP1) de Neisseria meningitidis, ainsi que tout peptide ou polypeptide natif ou de synthèse susceptible de remplir la même fonction, par exemple les peptides décrits dans la demande de brevet WO98/31393.

La liaison du polysaccharide à la protéine porteuse peut varier selon le polysacharide et la protéine utilisée. Il s'agit généralement de liaison covalente, pouvant faire intervenir un bras espaceur. Selon le mode de liaison mis en oeuvre, l'antigène obtenu que l'on appelle généralement conjugué, est un antigène dans lequel le polysaccharide est lié à la protéine porteuse par une seule fonction chimique (conjugués de type soleil ou néoglycoconjugués) ou par plusieurs fonctions (conjugués de type râteau ou pelote).

Le procédé de stabilisation d'une composition vaccinale liquide est charactérisé en ce que ladite composition vaccinale pouvant être plurivalente, il est possible d'ajouter à l'antigène constitué par le conjugué polysaccharide-protéine porteuse une ou plusieurs autres valences constituées également par un conjugué polysaccharide-protéine porteuse, ou par tout autre antigène de type différent. Parmi les autres valences susceptibles d'entrer dans la composition vaccinale selon l'invention, on peut citer notamment : la coqueluche, la polio, la diphtérie, le tétanos, l'hépatite (A,B,C...), la varicelle, les oreillons, la rougeole, la Dengue, l'encéphalite japonaise, la fièvre jaune, la rubéole, la grippe, la méningite, les pneumonies, ...etc.

Selon l'invention, ce procéde de stabilisation d'une composition vaccinale comprend, l'addition du tréhalose en quantité suffisante pour permettre le maintien au cours du temps de l'immunogénicité de l'antigène constitué par le conjugué polysaccharidique.

Le tréhalose ou α-D-glucopyranosyl α-D-glucopyranoside est un disaccharide connu pour son action protectrice des protéines lorsqu'elles sont soumises à des températures élevées, notamment lors d'opérations de séchage ou lyophilisation. Selon l'enseignement du document US 4 891 319 son action protectrice pourrait s'expliquer par une substitution des molécules d'eau par des molécules de tréhalose, les 2 composés comportant des fonctions OH.

Le tréhalose est également connu dans l'art antérieur comme protecteur de cellules.

De façon surprenante et sans que cela puisse se déduire des propriétés connues du tréhalose, il a maintenant été trouvé que ce composé permettait de conserver l'immunogénicité de compositions vaccinales, même dans le cas où celles-ci ne seraient pas soumises à une élévation de la température ou à un procédé de séchage.

Par contre, d'autres sucres testés connus pour avoir des propriétés proches du tréhalose, notamment le lactose, n'ont pas conduit à des résultats satisfaisants.

Selon une caractéristique particulière de l'invention, on a remarqué qu'une quantité de tréhalose comprise entre 3 et 12 %, et de préférence 5, était satisfaisante pour résoudre le problème de stabilité de la composition vaccinale. A cette concentration, aucune réaction de toxicité n'a été relevée.

Le tréhalose convenant aux fins de l'invention doit être un tréhalose de qualité pharmaceutique, sans qu'il soit cependant nécessaire qu'il présente un degré de pureté absolue. Le tréhalose fourni par la Société SIGMA sous la référence T9531 convient parfaitement bien.

L'addition du tréhalose peut être effectuée au début du procédé de fabrication ; il peut également être ajouté dans la formulation à la fin du procédé, seul ou en mélange avec les autres excipients.

Les exemples qui suivent illustrent plus particulièrement un mode de réalisation de l'invention.

### Exemple 1

On prépare 3 compositions vaccinales différentes en procédant de la manière suivante :
a- Fabrication de 4 solutions stock d'excipients
- solution de Tris Hydroxyl Amino Méthane 50 mM- saccharose 42.5 %
   Composition pour 1 litre:
   6.06 g Tris Hydroxyl Amino Méthane.
   425 g saccharose
   Eau PPI q.s.p.. 1 litre
- Solution de tréhalose à 20%
   Composition pour 400 ml
   Tréhalose: 80 g
   Eau PPI q.s.p.. 400 ml
   PH=7 ±0.1 après ajustement avec une solution de soude 2.5N
- Solution de chlorure de sodium 2M
   Composition pour 1 litre
   Chlorure de sodium: 117 g
   Eau PPI q.s.p.. 1 litre
- Solution de Tris Hydroxyl Amino Méthane 50 mM
   Composition pour 1 litre
   Tris Hydroxyl Amino Méthane: 6.06 g
   HCl 5N: 8.54 ml
   Eau PPI q.s.p.. 1 litre

b- Fabrication de 3 solutions d'excipients (A,B,C) à partir des 4 solutions stocks précédentes. Les volumes des solutions stocks utilisés sont indiqués dans le tableau ci dessous:

| **Solutions stock** | **Excipient 1** | **Excipient 2** | **Excipient 3** |
|---|---|---|---|
| Solution Tris Hydroxyl Amino Méthane 50 mM, saccharose 42.5 % | 72.6 ml | 0 | 0 |
| Solution tréhalose 20% | 0 | 100ml | 200 ml |
| Solution de chlorure de sodium 2M | 0 | 19ml | 0 |
| Solution Tris Hydroxyl Amino Méthane 50 mM | 0 | 72.6 ml | 72.6 ml |
| Eau PPI | q.s.p.. 363 ml | q.s.p.. 363 ml | q.s.p.. 363 ml |

c- Chaque solution d'excipient est stérilisée par filtration sur un filtre Millipack 60 de 0.22µm de seuil de coupure
d- Pour obtenir chacune des compositions vaccinales, on ajoute dans un flacon Schott de 500 ml stérilisé par autoclave, dans l'ordre : 290 ml de chacune des solutions d'excipients préparées puis 29.6 ml d'une composition contenant du PRP-T et du saccharose. Les flacons sont placés sous agitation 5 minutes à température ambiante puis 2 heures à 4°C.
e- Chaque composition est répartie dans des flacons peni en verre qui sont maintenus 6 mois à 25°C.

**La composition finale** de chaque formulation est résumée dans le tableau suivant :

| | **Composition A** | **Composition B** | **Composition C** |
|---|---|---|---|
| PRP-T (µg de polyoside /ml) | 20 20 | 20 20 | 20 20 |
| Tris Hydroxyl (mM) Amino Méthane (mM) | 10 mM | 10 mM | 10 mM |
| Saccharose (%) | 8.5% | 0.78% | 0.78% |
| Tréhalose (%) | 0% | 5% | 10% |
| NaCl (mM) | 0 | 0.095 mM | 0 |

### Exemple 2

On dispose de 5 lots de 8 souris femelles OF 1, d'un poids de 22 à 24 grammes. Les souris sont réparties par lots de 8. Chaque lot est utilisé pour tester une des compositions vaccinales A, B, ou C obtenues selon l'exemple 1, une composition vaccinale servant de témoin négatif (comprenant uniquement du PRP non conjugué) ainsi qu'une composition vaccinale servant de témoin positif qui est constituée du vaccin Act-Hib™ commercialisé par la Société PASTEUR MERIEUX Sérums et Vaccins.

On administre à chaque souris, par voie sous-cutanée, 0,5 ml de la composition vaccinale à tester correspondant à 2,5 µg de polyoside. Chaque souris reçoit une injection à J0 et une injection de rappel à J14.
Le sérum de chaque souris est prélevé à J0, J14 et J21.

### Exemple 3

Les sérums prélevés sont titrés par Radio Immuno-Assay. Les résultats obtenus sont exploités de la manière suivante :
- La moyenne géométrique est calculée à partir du titre des 8 sera.
- Le % de souris répondeuses (sérum ayant un titre >0.5) est déterminé.
- La différence des titres obtenue à J14 et J21 est calculée de manière à évaluer l'effet de l'injection de rappel.

Le produit est déclaré conforme lorsque les 3 conditions suivantes sont remplies :
- A J21, au moins 75 % des souris ont un titre >=0.5.
- La différence entre les titres obtenus à J14 et J21 est statistiquement significative.
- La différence de titre entre le produit testé et le témoin positif n'est pas statistiquement significative à J21.

Les résultats obtenus sont résumés dans le tableau suivant :

| | GMT à J14 | GMT à J21 | Conformité du Produit |
|---|---|---|---|
| Témoin Négatif | < 0,09 | 0,05 | - |
| Témoin Positif | < 0,1 | 1,3 | + |
| Composition A | 0,33 | 0,99 | - |
| Composition B | 0,13 | 1,6 | + |
| Composition C | 0,11 | 2,9 | + |

Ces résultats montrent que les compositions vaccinales selon l'invention conservent leur immunogénicité après stockage pendant 6 mois à 25°C.

Des tests effectués de la même façon sur des compositions stockées à 37°C, ont montré qu'une composition selon l'invention, comprenant 5 % de tréhalose, conservait son caractère immunogène même après 3 mois de stockage.

## Revendications

1. Procédé de stabilisation d'une composition vaccinale liquide comprenant au moins un antigène constitué par un polysaccharide lié à une protéine porteuse, **caractérisé en ce qu'**il consiste à ajouter à la composition vaccinale du tréhalose.

2. Procédé selon la revendication 1, **caractérisé en ce que** la quantité de tréhalose à ajouter est comprise entre 3 et 12 % en masse.

3. Procédé selon une des revendications précédentes **caractérisé en ce que** ledit polysaccharide est le polysaccharide capsulaire de Haemophilus influenzae type b ou Polyribosylribitol Phosphate.

4. Procédé selon une des revendications 1 ou 2, **caractérisé en ce que** ledit polysaccharide est un polysaccharide de Pneumocoque.

5. Procédé selon une des revendications 1 ou 2, **caractérisé en ce que** ledit polysaccharide est un polysaccharide de méningocoque.

6. Procédé selon une des revendications précédentes, **caractérisé en ce que** ladite protéine porteuse est l'anatoxine tétanique.

7. Procédé selon une des revendications précédentes, **caractérisé en ce que** ladite protéine porteuse est l'anatoxine diphtérique.

8. Procédé selon une des revendications précédentes **caractérisé en ce que** la quantité de tréhalose à ajouter est d'environ 5 %.

## Patentansprüche

1. Verfahren zur Stabilisierung einer flüssigen Impfstoffzusammensetzung, die wenigstens ein Antigen, das aus einem an ein Trägerprotein gebundenen Polysaccharid besteht, umfasst, **dadurch gekennzeichnet, dass** es darin besteht, der Impfstoffzusammensetzung Trehalose zuzufügen.

2. Verfahren gemäß Anspruch 1, **dadurch gekennzeichnet, dass** die zuzufügende Trehalosemenge zwischen 3 und 12 Masse-% liegt.

3. Verfahren gemäß einem der vorhergehenden Ansprüche, **dadurch gekennzeichnet, dass** besagtes Polysaccharid Kapselpolysaccharid von Haemophilus influenzae Typ b oder Polyribosylribitolphosphat ist.

4. Verfahren gemäß einem der Ansprüche 1 oder 2, **dadurch gekennzeichnet, dass** besagtes Polysaccharid ein Pneumokokken-Polysaccharid ist.

5. Verfahren gemäß einem der Ansprüche 1 oder 2, **dadurch gekennzeichnet, dass** besagtes Polysaccharid ein Meningokokken-Polysaccharid ist.

6. Verfahren gemäß einem der vorhergehenden Ansprüche, **dadurch gekennzeichnet, dass** besagtes Trägerprotein Tetanus-Anatoxin ist

7. Verfahren gemäß einem der vorhergehenden Ansprüche, **dadurch gekennzeichnet, dass** besagtes Trägerprotein Diphtherie-Anatoxin ist

8. Verfahren gemäß einem der vorhergehenden Ansprüche, **dadurch gekennzeichnet, dass** die zuzufügende Trehalosemenge etwa 5 % beträgt

## Claims

1. Method of stabilizing a liquid vaccine composition comprising at least one antigen consisting of a polysaccharide bound to a carrier protein, **characterized in that** it consists in adding trehalose to the vaccine composition.

2. Method according to Claim 1, **characterized in that** the quantity of trehalose to be added is between 3 and 12% by mass.

3. Method according to one of the preceding claims, **characterized in that** said polysaccharide is the capsular polysaccharide of Haemophilus influenzae type b or Polyribosylribitol Phosphate.

4. Method according to either of Claims 1 or 2, **characterized in that** said polysaccharide is a pneumococcal polysaccharide.

5. Method according to either of Claims 1 or 2, **characterized in that** said polysaccharide is a meningococcal polysaccharide.

6. Method according to one of the preceding claims, **characterized in that** said carrier protein is tetanus toxoid.

7. Method according to one of the preceding claims, **characterized in that** said carrier protein is diphtheria toxoid.

8. Method according to one of the preceding claims, **characterized in that** the quantity of trehalose to be added is about 5%.
